# EUROPEAN PATENT SPECIFICATION

(11) **EP 1 371 365 B1**
(45) Date of publication and mention of the grant of the patent: **21.09.2005**
(21) Application number: 03252828.3
(22) Date of filing: 06.05.2003
(51) Int. Cl.: A61K 9/16, A61K 9/10, A61K 47/48, B01J 13/14, A61K 51/00, A61K 39/12

(54) **Emulsion and subsequent aggregation process as methods for forming polymeric microspheres for biomedical human and veterinary administration**
Emulgierungs- und nachfolgender Aggregierungsprozess als Methoden zur Darstellung polymerer Mikrokügelchen für die biomedizinische Verwendung an Mensch und Tier
Les processus d'émulsification et d'aggrégation en tant que des méthodes de la formation des microsphères polymériques pour l'administration biomédicale chez les hommes et les animaux

(30) Priority: 07.05.2002 US 63656
(43) Date of publication of application: 17.12.2003
(73) Proprietor: Xerox Corporation, New York 14644 (US)
(72) Inventor: Burns, Patricia A., Milton, Ontario L9T 3B6 (CA); Mahabadi, Hadi K., Mississauga, Ontario L5M 1L7 (CA); Ziolo, Ronald F., Webster, NY 14580 (US); Patel, Raj D., Oakville, Ontario L6H 3L2 (CA)
(74) Representative: Skone James, Robert Edmund

(56) References cited:
- WO-A-01/70132
- US-A- 4 609 689
- US-A- 5 160 745
- US-A- 5 648 100
- US-A- 5 723 269
- US-A1- 2002 010 127
- US-B1- 6 238 702

## Description

The invention relates to polymeric microspheres, including magnetic and/or superparamagnetic polymeric microspheres, useful for biomedical applications. The polymeric microspheres of the present invention are generally of small size, about 1-15 microns, and are generally of a narrow particle or geometric size distribution. The present invention also relates to processes, particularly emulsion/aggregation polymerisation processes, useful for making such polymeric microspheres.

Polymeric microspheres, i.e., microspheres formed (at least in part) from polymer, have found a variety of uses in the medical and industrial areas. Furthermore, biodegradable polymers have been the subject of numerous studies in controlled drug delivery. As drug carriers, microspheres formed from biodegradable polymer(s) have the advantages of providing a large surface area, being easily injected, and not requiring removal after completion of drug release. When used as an injectable drug delivery device, it has been found that drug release rate and microsphere interaction with cells is strongly dependent on the size distribution of the microspheres.

US 5,160,745 describes a method for preparing biodegradable microspheres wherein a vinyl derivative of a biodegradable hydrophilic polymer which may be a polyester, a water soluble monovinyl monomer and a biologically active macromolecule are emulsified in water and co-polymerized. The microspheres have a particle size of 1-15µm in diameter.

US 4,609,689 relates to polymeric microbeads comprising a dispersion of polymeric particles as a first reactive functional group allowing the attachment of a second functional group.

US 5,648,100 details microspheres comprising a hydrophilic acrylic copolymer, a neutral hydrophilic acrylic monomer and a monomer coated with cell adhesion promoter. The microspheres have a size in the range from 10 to 2000µm in diameter and polymerization may be carried out in mass or in emulsion.

WO-A-01/70132 describes an injectable composition comprising biocompatible, biodegradable or non-biodegradable polymeric microspheres containing a therapeutic agent. The particle size range is 10 to 120µm. The microspheres may be prepared by inter alia suspension polymerisation and drop-by-drop polymerisation.

US 2002/010127 relates to controlled-release dosage forms containing an opioid agonist, an opiod antagonist and a controlled release material. Injectable formulations wherein the agents are in microparticles are described. The microparticles have a diameter in the range from 5 to 200µm.

US 5,723,269 describes a microparticle preparation. This preparation is produced by spraying a polymer solution containing a drug and a solution or dispersion of water-soluble inorganic salt, a water-soluble organic acid or a water-soluble salt of an organic acid from different nozzles and contacting them with each other in a spray drier.

US 6,238,702 relates to a controlled release dosage formulation. This formulation may be in the form of microparticles. The microparticles may, for example, be prepared using solvent evaporation, phase separation and fluidized bed coating. The microparticles preferably have a diameter in the range from 5 to 200µm.

Each of the known methods for forming polymeric microspheres has shortcomings that curtails utility of the formed-microspheres in various applications, and particularly when the methods are applied to the continuous production of uniformly sized microspheres, including biocompatible, biodegradable, drug-loaded microspheres.

A need exists for a simple and reliable method for producing uniformly-sized microspheres. It is desirable to be able to produce uniformly sized microspheres in a continuous fashion such that the size of the microspheres is easily controllable, the process is scaleable to large production, and allows the use of volatile solvents.

The invention provides methods suitable for preparing microspheres. The methods address the problems associated with the existing procedures, offer significant advantages when compared to existing procedures, and provide other advantages.

These and other objects are achieved by the invention, which provides an emulsion/aggregation process for producing polymeric microspheres. The polymeric microspheres exhibit a narrow particle or geometric size distribution, and have particle sizes making them useful for biomedical applications. Depending on the desired use, the polymeric microspheres can be subjected to a suitable treatment, which enables their use in the desired biomedical application.

In particular, the present invention provides a method of forming polymeric microspheres for biomedical applications, comprising:
forming polymeric microspheres by an emulsion/aggregation process from a precursor monomer species; and
treating said polymeric microspheres to attach a biomedical functional material to said polymeric microspheres,
wherein said polymeric microspheres have an average particle diameter of in the range from 1 to 15 microns with a narrow particle geometric size distribution.

In one embodiment, the emulsion/aggregation process comprises: forming a polymeric resin from said precursor monomer species; aggregating said polymeric resin into polymeric particles; coalescing said polymeric particles into polymeric microspheres; and optionally isolating said polymeric microspheres.

In another embodiment, the emulsion/aggregation process comprises: forming a polymeris resin from said precursor monomer species; forming an emulsion comprising said polymeris resin; coalescing said polymeric resin into polymeric microspheres; and optionally isolating said polymeric microspheres.

In a still further embodiment, the emulsion/aggregation process comprises;
providing a polyester resin formed from said monomeric species;
dispersing said polyester resin an aqueous media by heating in water, to provide a suspension of suspended particles of said polyester resin; homogenizing said suspension; aggregating said homogenized suspension by adding a cationic metal salt and optional additives to form aggregated and coalesced particles by heating the aggregates near, and preferably below, the glass transition temperature of the polyester resin, to form polymeric microspheres; and optionally isolating said polymeric microspheres.

In a still further embodiment, the emulsion/aggregation process comprises:
providing a polyester resin formed from said monomeric species;
dispersing said polyester resin in an aqueous media comprising an anionic surfactant, to provide a suspension of suspended particles of said polyester resin; homogenizing said suspension; aggregating said homogenized suspension by adding a cationic surfactant and optional additives to form aggregate; coalescing said aggregates by heating the aggregates above a glass transition temperature of the polyester resin, to form polymeric microsphres; and optionally isolating said polymeric microspheres.

The invention provides a process for forming microspheres, which includes forming suitable particles (polymeric microspheres) in an emulsion/aggregation process, and then subjecting the formed particles to suitable treatment, enabling use of the microspheres in the desired biomedical or other application.

In one embodiment, the particles are comprised of emulsion/aggregation (E/A) particles, i.e., particles prepared by the known emulsion/aggregation technique. Major advantages of E/A particles as the polymeric microspheres are that E/A particles have a very narrow particle size distribution, providing more uniform movement and properties of the particles, less likelihood of agglomeration during use, and minimum particle size classification subsequent to formation. The E/A processes are particularly suited for making such microspheres, as the processes are efficient in forming microspheres of the desired size range, with narrow particle size distribution. Another advantage of E/A particles is the ability to more easily incorporate additives, such as colorants (either conventional, fluorescent, or the like), magnetic and/or superparamagnetic materials, etc., into the microspheres. The EA process also provide a greater degree of flexibility in forming desired microspheres, as the E/A processes broaden the range of types of resins that can be used, and provide the ability to begin with resins that have functional groups in them or can easily be formed by reactions of the surface of the microsphere once formed.

Emulsion/aggregation processes for making particles, in which the particles are achieved via aggregation as opposed to particle size reduction, are well know. Such E/A processes generally include the steps of, e.g., emulsion, aggregation, coalescence, washing and drying. The E/A process is not limited in the use of certain polymers for toner particles, although polyesters and acrylic based polymers are convenient for use in the process, the use of polyesters having the further advantage of not requiring the use of any surfactants in making the particles. Fluoropolymers may also be used, these polymers showing excellent charge properties in hydrocarbons.

E/A particles may be made to have a suitably small size, for example on the order of from 0.5 to 20 microns or from 1 to 10 microns, with an excellent particle size distribution, compared to the scattered distribution typically exhibited from polymeric particles prepared by grinding techniques. E/A particles also can have specific surface treatments and shapes depending on the process conditions, which can be important parameters in various uses.

In embodiments of the invention, any suitable polymer material may be used to form the polymeric microspheres. The specific polymer used can depend, for example, on various considerations such as compatibility with the emulsion/aggregation process, compatibility with biological tissue, physical properties, chemical properties, and the like. Illustrative examples of polymer resins selected for the process and particles of the invention include polyesters, polystyrene, polyacrylate, polymethacrylate, polystyrene-butadiene, polystyrene-methacrylate, polystyrene-acrylate, mixtures thereof and the like.

According to embodiments of the invention, suitable polymer materials also include functionalized polymers, i.e., polymers that already incorporate functional groups, which functional groups will in turn be present and available for use in the formed polymeric microspheres.

According to one embodiment of the invention, an emulsion is prepared by agitating in water a mixture of one or more of an optional nonionic surfactant, an optional anionic surfactant and a monomer to form polymerized particles. Where more than one monomer is used, or where a monomer or polymer species is used as a seed for the polymerization process, polymerization of the monomers or polymers can take place in a manner to encapsulate or otherwise incorporate the monomer or polymer particles by heating from ambient temperature to 80°C. Emulsion sized resin particles are produced having a volume average diameter of from 0.02 to 1.2 microns specifically including all sub-ranges and individual values within the range of 0.02 to 1.2 microns. The resulting resin emulsion, which typically contains from 20% to 60% solids, is then preferably diluted with water to about 15% solids.

Next, one or more optional additives can be added to the resin emulsion, to be incorporated into the desired polymeric particles. For example, suitable additives can include colorants, magnetic materials, superparamagnetic materials, bioactive agents etc.

For example, one or more colorants, such as pigments or dyes, can be added to the resin emulsion in an amount less than or equal to 65% of the particle solids and preferably from 0.5% to 65% of particle solids. The colorants may be pretreated so as to bind resin particles of the invention thereto. Alternatively, the colorants may be encapsulated by the resin particles in whole or in part. The resulting mixture may optionally be dispersed utilizing a Brinkman or IKA homogenizer.

Further, one or more bioactive agents, such as medicaments, can be added to the resin emulsion in an amount less than or equal to 65% of the particle solids and preferably from 0.5% to 65% of particle solids. Like the colorants, the bioactive agents may be pretreated so as to bind resin particles of the invention thereto. Alternatively, the bioactive agents may be encapsulated by the resin particles in whole or in part. The resulting mixture may optionally be dispersed utilizing a Brinkman or IKA homogenizer.

When such additives are incorporated into the resin emulsion, optional flocculation of the emulsion can be conducted to assist in the polymeric microsphere production. The optionally flocculated resin-additive mixture is then suitably homogenized, for example at from 2000 to 6000 revolution per minute, to form statically bound aggregate composite particles, which are then heated at a suitable temperature of, for example, from 60 °C to 95 °C and for a suitable duration of time of, for example, 60 to 600 minutes, to form polymeric particles (microspheres) of the controlled size with narrow size distribution. According to the invention, the polymeric microspheres can have a suitable volume average diameter of, for example, from 0.5 to 25 microns, or from 1 to 15 or 20 microns, or from 2 or 3 to 10 or 15 microns. The polymeric microspheres have a geometric standard distribution of less than 1.3, preferably less than 1.25 or less than 1.20.

As mentioned above, any suitable monomer or polymer species may be used, as desired. Specific examples of nonionic monomers include styrene, alkyl substituted styrenes, halogenated styrenes, halogenated alkyl substituted styrenes and the like.

Examples of additional useful monomers include nonionic diolefinic or diene monomers such as butadiene, substituted butadienes, isoprene, mycerine, alkyl substituted isoprene, mixtures thereof and the like.

As desired, and based on the intended use of the polymeric microspheres, one or more monomers or polymers can be used in the polymerization process. When so used, the resultant resin latex can include homopolymers, copolymers, or higher order polymers (terpolymers and the like). Where copolymers or terpolymers are present, such polymers can be block, graft, random, or the like, or combinations thereof.

In general, useful colorants or pigments include carbon black, magnetite, or mixtures thereof; cyan, yellow, magenta, or mixtures thereof; or red, green, blue, brown, or mixtures thereof. Typical useful colorants or pigments are present in an effective amount of, for example, from 1 to 65wt%, from 1 to 25wt% or from 3 to 10wt%. Furthermore, in embodiments of the invention, it may be possible to utilize lesser amounts of the colorants, such as from 0.1 to 10wt% or from 0.5 to 5wt%.

Additional useful colorants include pigments in water based dispersions such as those commercially available from Sun Chemical and Clariant.

Other useful colorants include magnetites and surface treated magnetites;

Examples of the surfactant, which can be added to the aggregates before coalescence is initiated, can be anionic surfactants or an effective amount of the anionic or nonionic surfactant utilized in the coalescence to primarily stabilize the aggregate size against further growth with temperature is, for example, from about 0.01 to10wt%, and preferably from 0.5 to 5wt% of the reaction. Additional methods of stabilizing aggregate size include raising pH of the emulsion above 6, such as through the addition of sodium hydroxide or potassium hydroxide.

Dyes that are invisible to the naked eye but detectable when exposed to radiation outside the visible wavelength range (such as ultraviolet or infrared radiation) are also suitable:

In addition, suitable colorants that can be used in the invention include one or more fluorescent colorants, which can be pigments, dyes, or a mixture of pigments and dyes.

Suitable fluorescent dyes include rhodamines, fluoresciens, coumarins, napthalimides, benzoxanthenes, acridines, azos, and the like.

As mentioned above, magnetic and/or superparamagnetic materials can also be used. Such magnetic materials can give the polymeric microspheres magnetic and/or superparamagnetic properties, for colorant properties, or the like. Suitable magnetic and/or superparamagnetic materials that can be used in the invention include magnetites, ferrites, and the like. Examples of suitable materials include a mixture of iron oxides, barium ferrite powder, strontium ferrite powder, barium-strontium ferrite powder, SmCo₅-based powder, Sm₂Co₁₇-based powder, Nd₂Fe₁₄B-based powder, Sm₂Fe₁₇N₃-based powder, (NdDy)₁₅Fe₇₉B₆, alloys of 33Ne 66Fe 1B, an Nd-Fe-B-based quenched magnetic powder, ferrite particles, and the like. Any other suitable magnetic and/or superparamagnetic material can also be used. The magnetic and/or superparamagnetic material can be present in the polymeric microspheres in any effective amounts, such as from 10wt% to 75wt% of the polymeric microspheres. Preferably, the magnetite and/or superparamagnetic is present in an amount of from 30 to 55wt% of the polymeric microspheres.

Examples of useful chain transfer agents that can be included in the processes of the invention include, dodecanethiol, carbon tetrabromide and the like, which can be used to control the molecular weight properties of the polymer when emulsion polymerization is carried out. An effective concentration of a chain transfer agent that is generally employed is, for example, from 0.005 to 10wt%, or from 0.01 to 5wt%, or from 0.1 to 3wt%.

Examples of useful optional free radical initiators include azo-type initiators, An effective quantity of an initiator is generally within the range of 0.1 to 10wt% of the reaction mixture.

In addition to the above specifically identified monomers and polymers, the processes of the invention are also applicable to polymeric microspheres made from polyester resins, such as sulfonated polyester resins.

In such polyester emulsion/aggregation processes, the polyester can be obtained from the melt esterification of dicarboxylic acid or diester components with diol component, and optionally a sulfonated difunctional monomer, and using a polycondensation catalyst. The dicarboxylic acid and/or diester components are generally present in an amount of from 42 to 49.5 mol% of the polyester; the diol component is generally present in an amount of 50 mol% of the polyester resin; and the sulfonated difunctional monomer, when present, is generally present in an amount of from 0.5 to 8 mol% of polyester. The polycondensation catalyst is generally present in an amount of from 0.01 to 0.1 mol% of the polyester.

The emulsion/aggregation process for forming such polyester polymeric microspheres generally comprises obtaining or forming the polyester resin, followed by (a) dissipating the polyester resin in water by heating at from 60°C to 120°C with mixing for from 1 minute to 1 hour thereby generating suspended polyester particles of from 0.01 to 2 microns in average particle diameter; (b) subsequently adding to the resulting emulsion suspension an optional pigment or other additive dispersion, such as in an aqueous mixture containing a counterionic metal salt, and resulting in the aggregation and coalescence of resin particles and optional pigment or additive of from 3 to 21 microns thereby providing polymeric microspheres with a desired average particle volume diameter; and (c) cooling the mixture to ambient temperature, washing with water from three to six times, and drying the product by known methods such as fluid bed dryer.

If desired, the polymeric microspheres may optionally include known charge additives in effective suitable amounts of, for example, from 0.1 to 5wt%. Such charge additives are well known for use in electrostatographic imaging toner compositions.

Once the polymeric microspheres are formed, they can be isolated from the reaction mixture by any suitable means. Suitable isolation methods filtration, particle classification, and the like.

Alternatively, the formed polymeric microspheres can be washed and dried by conventional means.

The polymeric particles may subsequently be subjected to an optional chemical surface treatment. For example, the polymeric particles may be subjected to any desirable surface treatment to alter the chemical and/or physical properties of the particle, such as hydrophobicity, hydrophilicity, surface charge, and the like, or to attach or alter functional groups present on the surface of the microspheres.

Surface additives that can optionally be added to the polymeric microspheres include, for example, metal salts, metal salts of fatty acids, colloidal silicas, metal oxides like titanium, mixtures thereof and the like, usually present in an amount of from 0.1 to 2wt%. Additional additives include zinc stearate in an amount of from 0.1 to 2% and silica in amounts of from 0.1 to 5% which can be added during the aggregation process or blended into the formed product.

The formed polymeric microspheres of the invention can also or alternatively be surface halogenated, partially or wholly, for example 100 percent, to convert olefinic double bonds by an electrophilic addition reaction in the surface polymer chain backbone and pendant groups into the corresponding halogenated hydrocarbon functionality. Surface halogenation of the polymeric microspheres affords further control of the variety of rheological properties that may be obtained from the copolymer resins. Surface halogenation is accomplished with a gaseous mixture or liquid solution of an effective amount of from 0.01 to 5 double bond molar equivalents of halogen gas or halogen liquid dissolved in water, or an organic solvent, for example, chlorine gas, liquid bromine, or crystalline iodine dissolved in a solvent, such as an aliphatic alcohol, like ethanol which does not dissolve or substantially alter the size or shape of the polymeric microspheres.

When more reactive halogens such as fluorine are used, an inert carrier gas may be selected as a diluent, for example, from 0.1 to 98 percent by volume of the inert gas relative to the reactive halogen gas, to moderate the extent of reaction, and the temperature and control corrosivity of the halogenation-encapsulation process.

A number of equally useful halogenating agents are known that afford equivalent reaction products with olefinic double bonds as the aforementioned diatomic halogens

The aforementioned halogenation can be considered an addition reaction. That is, for example, the halogen reacts with, and diffuses into the polymer resin, whereby a shell thereof is formed. The shell can be of various effective thicknesses; generally, however, the shell is of a thickness of from about 1 micron or less, and more specifically from 0.1 to 1 micron, in embodiments. Typical amounts of halogen consumed include, for example, from 0.1 to 1 gram of halogen per 100 grams of polymer resin.

Other functional groups can be present on the surface of the polymeric microspheres, either by being present in the polymer material itself resulting from the polymerization process, or by surface modification of the formed microspheres. Various methods for forming such functional groups are known in the art. Such functional groups can be modified to enable ready attachment of biological or other materials. Alternatively, the functional groups can be reacted with materials that in turn may act as linkages to biological materials, ligands or other materials.

For example, hydroxyl groups can be reacted with sulfonyl chlorides to enable nucloeophilic attack by amines, alcohols, and the like. The amine portion of a protein can react and lead to attachment of the protein to the surface of the microsphere. Alcohols can undergo esterification to attach reactive functional groups, to which biological or other materials can be attached. Primary amines can be converted to amides, acids, or sulfonates, which can possess suitable functional groups for further manipulation. Chloromethyl groups can be converted to amines, ethers, aldehydes and thiols, which can be linked to biological or other materials by known methods. Carboxylic acid can be converted to esters or amides and can often directly react with proteins. Ester groups can undergo transesterification. Amino groups can couple to proteins with glutaraldehyde. Epoxy groups introduced by any of the previous manipulations (or others) can be readily reacted with biological materials to enable attachment. Other suitable of include making the particles more hydrophobic

Of course, two or more different surface treatments or modifications can be performed on the same microsphere, if desired. Such multiple surface treatment or modification can be advantageous, for example, to enable attachment of multiple different materials or to provide multiple functionalities to the microspheres.

The polymeric microspheres prepared according to the invention can be used for a variety of uses. The microspheres are especially useful for biological and medical purposes. Accordingly, the polymeric microspheres of the invention are preferably biocompatible. By biocompatible is meant that the polymeric microspheres and additives are not destructive to healthy living tissues, at least beyond any desired intended effect, over the lifetime that the polymeric microspheres are in contact with or proximity to such biological tissues. However, where the polymeric microsphers are to be used *ex vivo*, biocompatibility is unnecessary.

The microspheres described herein can be used as size standards for analytical purposes.

In a preferred embodiment of the invention, the microspheres are composed of biodegradable, biocompatible polymers and contain a bioactive agent dispersed therein. The bioactive agent may be dissolved within the polymer phase or dispersed as discrete solid particles.

Microspheres of the present invention can also be used for embolization, including preoperative embolization of hemangioblastomas.

The invention also contemplates the use of microspheres to measure the rate of blood flow.

Uniformly sized microspheres can also be used to deliver a bioactive agent to a subject in need of treatment. Such therapeutic microspheres can be produced either by incorporating a bioactive drug within the microspheres, or by binding a bioactive drug to the surfaces of microspheres. Examples of suitable bioactive agents include anti-proliferative agents, steroids, analgesics, narcotic antagonists, antibiotics, antifungals, anti-histamines, anti-asthmatics, beta-blockers, and anti-cancer agents. In addition, radioactively-labeled microspheres can be used for therapeutic purposes

A suitable bioactive agent for incorporation within a microsphere should have a solubility parameter close to that of the polymer used to make the microsphere. In this way, the same solvent can be used to dissolve the bioactive agent and the polymer to produce a homogeneous solution.

Such a preference for solubility does not apply, however, to therapeutic microspheres comprising a bioactive agent bound to the surface. For example, polypeptides, such as pharmacologically active peptides, antigens, and antibodies, are suitable for binding to microsphere surfaces. As an illustration, microspheres can be produced that bear an infectious agent antigen for vaccination.

Microspheres can also be produced that comprise bioactive agents incorporated within the microsphere and bound to the surface. For example, antibodies or antibody fragments can be bound to a microsphere surface to target delivery of another bioactive agent contained within the microsphere.

In addition, microspheres can be incorporated into a second polymer. That is, uniformly sized microspheres can be dispersed throughout a gel or a viscous solution, or dispersed throughout a solid, biodegradable polymer scaffold.

Therapeutic microspheres can be delivered to a subject via transdermal, oral, nasal, pulmonary, ocular, or parenteral routes. Examples of parenteral routes include intramuscular, intra-tumoral, intra-arterial, and intravenous administration. Since therapeutic microspheres can be used for both human therapy and veterinary purposes, suitable subjects include both humans and non-human animals.

Microspheres comprising a bioactive agent can also be used in a variety of agricultural applications.

Controlled release systems for the release of macromolecules from a polymer matrix have been proven effective for many applications. For example poly(ethylene vinylacetate), (EVA) has been studied for this use, and may be used as the polymer in the compositions according to the invention. EVA has been used in delivery systems for various macromolecules such as herapin, enzymes, and antigens. The mechanism of controlled release of macromolecules from non-degradable polymers has been shown to involve aqueous diffusion of the macromolecule through a network of interconnecting channels that arise within the polymer at the time of incorporation of the macromolecule.

To achieve such uses, the polymeric microspheres can be processed to attach suitable biological or medical materials to the surfaces of the microspheres. Such biological materials include materials that serve specific therapeutic, diagnostic, analytical, or experimental purposes and include, radioactive, biological, and ligand materials. The biological materials can be attached to the surface of the microspheres by, covalent bonding, complexation, physical adsorption, physical absorption etc.

In the case of radio-labeled or radioactive polymeric microspheres, the radioactive component can be incorporated into the microsphere or can be applied to the formed microsphere, as desired. When incorporated into the microsphere itself, it is appropriate to add the radioactive material to the emulsion during the production process. The radioactive component can, as desired, be a material that is itself radioactive, or it can be a radioactive precursor material that becomes radioactive upon exposure to a suitable initiating source.

In a preferred embodiment of the invention, the radioactive constituent of the microspheres is chosen so that when administered to the patient, the radioactive microspheres emit a therapeutic intensity and amount of short-range (e.g., a penetration of the tissue on the order of several millimeters or less) beta or gamma radiation, but will not emit a significant amount of unwanted beta or gamma radiation that could have a negative impact on healthy tissue surrounding the cancerous or tumor bearing tissue. In this regard, it is preferred that the components of the polymeric microspheres be selected so that the radiation emitting radioisotopes are the only constituent isotopes that may emit a significant amount of radiation beyond a relatively short period of time, e.g., on the order of 1 week or less.

The remaining constituent elements of the polymeric microspheres of the invention are also preferably chosen so that the microsphere does not contain any significant amount of elements that have a large cross-section for neutrons. An example of one such element that has a large cross-section for neutrons is boron, which has a cross-section of 3837 barns. Preferably, the microspheres do not contain a significant amount of any elements having a cross-section for neutrons greater than 200 barns.

In the case of polymeric microspheres containing a biological material, one or more biological materials can be incorporated into the microsphere or can be applied to the formed microsphere, as desired. When incorporated into the microsphere itself, it is appropriate to add the bioactive material to the emulsion during the production process.

As used herein, a bioactive material, or bioactive agent, refers to materials that exhibit therapeutic effects when applied or otherwise exposed to biological tissues, organs, fluids or the like. Bioactive materials, or bioactive agents, thus refers to any such materials that include medicaments, as well as other active agents that effect such biological tissues, organs, fluids or the like.

Examples of such bioactive materials thus include antibiotics, antimicrobials, antiseptics, bacteriocins, bacteriostats, disinfectants, steroids, anesthetics, fungicides, anti-inflammatory agents, antibacterial agents, antiviral agents, antitumor agents, growth promoters, and mixtures thereof. Preferable bioactive materials are USP approved or, monographed.

Likewise, in the case of polymeric microspheres including one or more ligands, the ligand can be incorporated into the microsphere or can be applied to the formed microsphere, as desired. Preferably, when ligands are to be included in or on the microspheres, the ligand can be added to the material to the emulsion during the production process, but it is preferably instead added onto the surface of the microspheres after the microspheres are formed.

Suitable ligands that can be attached to the polymeric microspheres according to the invention include any of the known ligands. Thus, for example, suitable ligands include, proteins, enzymes, analytes, antigens, antibodies etc. Such materials can be attached to the microsphere surface, such as through absorption or adsorption.

Suitable antigen may be derived from a cell, bacterium, virus particle, or a portion thereof. The antigen may be a protein, peptide, polysaccharide, glycoprotein, glycolipid, nucleic acid, or a combination thereof, which elicits an immune response in an animal, including mammals, birds, and fish. The immune response may be a humoral immune response or a cell-mediated immune response. In some embodiments it may be desirable to include an additional adjuvant with the antigen. Of course, other suitable antigens could also be used

Examples of preferred antigens include viral proteins such as influenza proteins, human immunodeficiency virus HIV proteins, Haemophilus influenza proteins, hepatitis B proteins, and bacterial proteins and lipopolysaccharides such as gram negative bacterial cell walls and Neisseria gonorrhea proteins.

Whether the microspheres of the invention are essentially void-free, microshells, or have a plurality of hollow cells, it is preferred that the microspheres be substantially spherical, i.e., there are no sharp edges or points that would cause the microsphere to lodge in a location other than that desired. In this context, elipsoidal and other similarly shaped particles that do not have sharp edges or points would be considered to be substantially spherical in shape.

### Example 1

1. Aggregation of Styrene/Butylacrylate/Acrylic Acid Latex:
2. Pigment dispersion: 7 grams dry pigment SUN FAST BLUE™ and 1.46 grams of cationic surfactant SANIZOL B-50™ are dispersed in 200 grams of water at 4,000 rpm using a blender.

A polymeric latex is prepared by emulsion polymerization of styrene/butylacrylate/acrylic acid (82/18/2 parts) in a nonionic/anionic surfactant solution (3 percent) as follows. 352 grams styrene, 48 grams butylacrylate, 8 grams of acrylic acid, and 12 grams dodecanethiol are mixed with 600 milliliters of deionized water in which 9 grams sodium dodecyl benzene sulfonate anionic surfactant (NEOGEN R™ which contains 60 percent of active component), 8.6 grams of polyoxyethylene nonyl phenyl ether-nonionic surfactant (ANTAROX 897™--70 percent active component), and 4 grams ammonium persulfate initiator are dissolved. The emulsion is polymerized at 70°C for 8 hours. The resulting latex contains 60 percent water and 40 percent solids of primarily polystyrene/polybutyacrylate/polyacrylic acid 82/18/2 resin; Tg 53.1°C; M_{w} =20,000, and Mₙ =6,000. The particle size of the latex is 160 nanometers.

### 2. Preparation of Microsphere Size Particles--11.7 Percent of Solids

Comprising the Above Resin Particles (95 Percent) and Pigment Particles (5 Percent) and Sheared)

Preparation of the aggregated particles: 208.5 grams of the above prepared SUN FAST BLUE™ dispersion are added to 300 milliliters water containing 1.5 grams cationic surfactant alkylbenzyldimethyl ammonium chloride (SANIZOL B-50™). This dispersion is simultaneously added with 325 grams of the above prepared latex into SD41 continuous stirring device containing 300 grams water. The pigment dispersion and the latex are well mixed by the continuous pumping through the shearing chamber operating at 10,000 rpm for 8 minutes. A homogeneous blend is obtained, which is then transferred into a kettle placed in a heating mantle, and equipped with mechanical stirrer and temperature probe. The temperature in the kettle is raised from room temperature to 45°C where the aggregation is performed for 2 hours, while stirring at 400 rpm. Aggregates with a particle size (average volume diameter) of 4.7 and GSD of 1.20 are obtained.

Coalescence of aggregated particles: after the above aggregation, 55 milliliters of 20 percent anionic surfactant (NEOGEN R™) are added and the stirring speed is reduced from 400 rpm to 150 rpm. The temperature in the kettle is raised from 45°C to 85°C at 1°C/minute. Aggregates of latex and pigment particles are coalesced at 85°C for 4 hours. After 30 minutes of heating at 85°C, a microsphere particle size of 4.7 microns average volume diameter, and a GSD of 1.20 is obtained. After 4 hours of heating, microsphere particles of 4.6 microns (average volume diameter throughout) with a 1.21 GSD are obtained, indicating that both the particle size and GSD are retained during the coalescence step.

The resulting polymeric microspheres are comprised of poly(styrene-cobutylacrylate-co-acrylic acid), 95 percent, and cyan pigment, 5 wt% of particles. The polymeric microspheres are washed by filtration using hot water (50°C) and dried on a freeze dryer. The yield of dry polymeric microspheres is 95 percent.

### Example 2

The procedures of Example 1 are repeated, except that the cyan-colored pigment dispersion is replaced by a dispersion of MAPICO BLACK™ to give a magnetic material. The polymer latex is formed as in Example 1, and is mixed with 15wt% of the magnetic material dispersion. The microsphere size particles are prepared and coalesced as described above.

The resulting magnetic polymeric microspheres are comprised of poly(styrene-co-butylacrylate-co-acrylic acid), 85 percent, and MAPICO BLACK™, 15wt% of particles. The polymeric microspheres are then washed by filtration using hot water (50°C) and dried on a freeze dryer.

### Example 3

### 1. Preparation of aspartic acid containing polyester-amine resin:

A sodiosulfonated random polyester-amine resin containing pendant amine groups and comprised of, on a mol% basis, approximately 0.415 mole terephthalate, 0.05 mole aspartic acid, 0.35 mole sodium sulfoisophthalate, 0.375 mole 1,2-propanediol, 0.025 mole diethylene glycol, and 0.100 mole dipropylene glycol is prepared as follows.

In a one liter Parr reactor equipped with a bottom drain valve, a double turbine agitator, and a distillation receiver containing a cold water condenser are charged 368.6 grams dimethylterephthalate, 52 grams sodium dimethylsulfoisophthalate, 13.31 grams aspartic acid, 285.4 grams 1,2-propanediol, 285.4 grams dipropylene glycol, 26.025 grams diethylene glycol (1 mole excess of glycols), and 0.8 gram butyltin hydroxide oxide as the catalyst. The reactor is heated to 165°C with stirring for 3 hours whereby 115 grams of distillate are collected in the distillation receiver, and which distillate is comprised of 98vol% of methanol and 2vol% 1,2-propanediol. The mixture is then heated to 190°C over a one hour period, after which the pressure is slowly reduced from atmospheric pressure to 260 Torr over a one hour period, and then reduced to 5 Torr over a 2 hour period with the collection of approximately 122 grams of distillate in the distillation receiver, and which distillate is comprised of approximately 97 percent by volume of 1,2-propanediol and 3 percent by volume of methanol. The polymer resulting is discharged through the bottom drain valve of the reactor onto a container cooled with dry ice to yield 460 grams of a 3.5 mol% amine containing sulfonated-polyester resin, copoly(1,2-propylene-ethyleneoxyethyleneterephthalate)-copoly(copoly(1,2- propylene-ethyleneoxyethylene-sodio 5-sulfoisophthalate-copoly(copoly(1,2-propylene-ethyleneoxyethylene-aspart ate). The properties of the sulfonated-amine containing polyester resin are Tg= 54.1°C (onset), softening point = 150.8°C, Mn= 3,500g/mol, Mw 5,660g/mol.

### 2. Preparation of Microsphere Size Particles:

Polymeric microspheres are prepared from and Containing 96wt% of the sulfonated polyester amine, and 4wt% of cyan 15:3 pigment, as follows.

To a 3 liter reaction vessel equipped with a mechanical stirrer is added the sulfonated polyester amine resin (250 grams), into water (2 liters) at 80°C to yield an emulsion with particles therein, and wherein the particle diameter size is 70 nanometers. The resulting emulsion is cooled down to 50°C to 60°C, and 23 grams FLEXIVERSE CYAN 15:3 pigment dispersion, available from Sun Chemical, and comprised of 45wt% of the cyan pigment in water, such that the total amount of pigment in the polymeric microspheres is 4wt%, is then added. The resulting mixture is heated to 56°C, and to this is added 500 milliliters of a 5 percent zinc acetate aqueous solution at a rate of 1 milliliter per minute. The toner particle size of the mixture is monitored until it reaches a size (volume average diameter) throughout of 6 microns, after which the reaction mixture is quenched with 500 milliliters of cold water (2°C). The contents of the reaction vessel are filtered through a 25 micron screen, and the toner product is filtered, redispersed in 2 liters of water for one hour, refiltered a second time, reslurried in 2 liters of water again, refiltered a third time and freeze dried to yield 205 grams of polymeric microspheres with a particle size of 6 microns and GSD of 1.18.

### Example 4

The procedures of Example 3 are repeated, except that the cyan-colored pigment dispersion is replaced by a dispersion of MAPICO BLACK™ to give a magnetic material. The polymer latex is formed as in Example 3, and is mixed with 15wt% of the magnetic material dispersion. The microsphere size particles are prepared and coalesced as described above.

The resulting magnetic polymeric microspheres are comprised of aspartic acid containing polyester-amine resin, 85 percent, and MAPICO BLACK™, 15wt% of particles. The polymeric microspheres are then washed by filtration using hot water (50°C) and dried on a freeze dryer.

### Example 5

Polymeric microspheres are prepared as in Examples 1-4, except that a small amount of a combination of yttrium-89 and phosphorus-31, which upon irradiation for a short period of time with an intense thermal neutron flux generated by a nuclear fission reactor, generates yttrium-90 and phosphorus-32, is added to the respective pigment or magnetite dispersions.

The resultant polymeric microspheres include the radioactive precursor components in an amount to be suitable for radio-labeling purposes. The polymeric microspheres exhibit the same particle size and particle size distribution as described above for Examples 1-4.

### Example 6

Polymeric microspheres are prepared using the processes of Examples 1-4. Following formation of the polymeric microspheres, the microspheres are processed to adsorb an antigen, namely an influenza protein, to the surface of the microspheres.

The antigen attaches well to the microsphere and provides a product suitable for administration to a patient.

## Claims

1. A method of forming polymeric microspheres for biomedical applications, comprising:
forming polymeric microspheres by emulsion polymerization of a precursor monomer species to form a polymeric resin followed by an aggregation step to form polymeric microspheres; and
treating said polymeric microspheres to attach a biomedical functional material to said polymeric micropsheres,
wherein said polymeric microspheres have an average particle diameter in the range from 1 to 15 microns with a narrow particle geometric size distribution.

2. The method of claim 1 wherein said narrow particle geometric size distribution is less than 1.25.

3. The method of claim 1 wherein said polymeric microspheres are formed from a polymer selected from the group comprising polyesters and acrylic based polymers.

4. The method of claim 3 wherein said polyester is obtained from melt esterification of dicarboxylic acid or diester components with diol components in the presence of a polycondensation catalyst.

5. The method of claim 1 wherein the biomedical functional material is attached to said polymeric microspheres by at least one of covalent bonding, complexation, physical adsorption and physical absorption.

6. The method of claim 1 wherein the polymeric microspheres are formed from said precursor monomer species and one or more additives.

7. The method of claim 1, further comprising surface treating said polymeric microspheres subsequent to said forming step but prior to said treating step to alter a chemical property of a surface of said polymeric microspheres.

8. The method of claim 7 wherein said chemical property is selected from the group consisting of hydrophobicity, surface charge, and the presence of functional groups.

9. The method of claim 1 wherein the polymeric microspheres are biocompatible.

10. The method of claim 1 wherein the polymeric microspheres are biodegreadable.

11. The method of claim 1 wherein the polymeric microspheres are non biodegradable.

12. The method of claim 1 wherein the aggregation step comprises aggregating said polymeric resin into polymeric particles;
coalescing said polymeric particles into polymer microspheres; and
optionally isolating said polymeric microspheres.

13. The method of claim 1 wherein the aggregation step comprises forming an emulsion comprising said polymeric resin;
coalescing said polymeric microspheres; and
optionally isolating said polymeric microspheres.

14. The method of claim 1 wherein the polymeric resin is a polyester resin, prior to aggregation the polyester resin is dispersed in an aqueous media optionally comprising a surfactant, to provide a suspension of suspended particles of said polyester resin and the suspension is homogenized; and wherein the aggregation step comprises aggregating and coalescing said homogenized suspension by adding a cationic metal salt and optional additives, and heating the aggregates, to form polymeric microspheres wherein said heating is conducted at or near a glass transition temperature of the polyester resin.

## Patentansprüche

1. Verfahren zur Bildung von polymeren Mikrokügelchen für biomedizinische Anwendungen, wobei das Verfahren die folgenden Schritte umfasst:
Bildung von polymeren Mikrokügelchen durch Emulsionspolymerisation aus Monomervorstufen, um ein Polymerharz zu bilden, gefolgt von einem Aggregationsschritt zur Bildung von polymeren Mikrokügelchen, und
Behandlung der polymeren Mikrokügelchen, um ein biomedizinisches, funktionelles Material an die polymeren Mikrokügelchen.zu binden,
wobei die polymeren Mikrokügelchen einen durchschnittlichen Partikeldurchmesser in dem Bereich von 1 bis 15 Mikron mit einer engen geometrischen Partikelgrößenverteilung besitzen.

2. Verfahren nach Anspruch 1, wobei die enge geometrische Partikelgrößenverteilung weniger als 1,25 beträgt.

3. Verfahren nach Anspruch 1, wobei die polymeren Mikrokügelchen aus einem Polymer gebildet werden, das aus der Gruppe ausgewählt ist, die Polyester und Polymere auf Acrylbasis umfasst.

4. Verfahren nach Anspruch 3, worin der Polyester durch Schmelzveresterung von Dicarbonsäure- oder Diester-Komponenten mit Diolkomponenten in Gegenwart eines Polykondensationskatalysators erzielt wird.

5. Verfahren nach Anspruch 1, worin das biomedizinische, funktionelle Material wenigstens durch kovalente Bindung, Komplexbildung, physikalische Adsorption oder physikalische Absorption an die polymeren Mikrokügelchen gebunden wird.

6. Verfahren nach Anspruch 1, wobei die polymeren Mikrokügelchen aus den Monomervorstufen und einem oder mehreren Additiven gebildet werden.

7. Verfahren nach Anspruch 1, das ferner eine Oberflächenbehandlung der polymeren Mikrokügelchen umfasst, die nach dem Bildungsschritt, aber vor dem Behandlungsschritt durchgeführt wird, um eine chemische Eigenschaft einer Oberfläche der polymeren Mikrokügelchen zu verändern.

8. Verfahren nach Anspruch 7, wobei die chemische Eigenschaft aus der Gruppe ausgewählt wird, die aus Hydrophobie, Oberflächenladung und der Gegenwart von funktionellen Gruppen besteht.

9. Verfahren nach Anspruch 1, wobei die polymeren Mikrokügelchen biokompatibel sind.

10. Verfahren nach Anspruch 1, wobei die polymeren Mikrokügelchen biologisch abbaubar sind.

11. Verfahren nach Anspruch 1, wobei die polymeren Mikrokügelchen nicht biologisch abbaubar sind.

12. Verfahren nach Anspruch 1, wobei der Aggregationsschritt die Aggregation des polymeren Harzes in polymere Partikel,
die Koaleszenz der polymeren Partikel in polymere Mikrokügelchen, und
die optionale Isolierung dieser polymeren Mikrokügelchen umfasst.

13. Verfahren nach Anspruch 1, wobei der Aggregationsschritt die Bildung einer Emulsion, welche das polymere Harz umfasst,
die Koaleszenz der polymeren Mikrokügelchen, und
die optionale Isolierung der polymeren Mikrokügelchen umfasst.

14. Verfahren nach Anspruch 1, wobei das polymere Harz ein Polyesterharz ist, vor der Aggregation das Polyesterharz in einem wässrigen Medium, das optional ein oberflächenaktives Mittel umfasst, dispergiert wird, um eine Suspension der suspendierten Partikel des Polyesterharzes bereitzustellen, und die Suspension homogenisiert wird, und wobei der Aggregationsschritt die Aggregation und die Koaleszenz der homogenisierten Suspension umfasst, indem ein kationisches Metallsalz und optionale Additive zugegeben und die Aggregate erhitzt werden, um polymere Mikrokügelchen zu bilden, wobei dieses Erhitzen bei oder nahe an einer Glasübergangstemperatur des Polyesterharzes durchgeführt wird.

## Revendications

1. Procédé de formation des microsphères polymères pour des applications biomédicales, comprenant :
la formation de microsphères polymères par une polymérisation en émulsion d'une variété de monomère précurseur pour former une résine polymère suivie d'une étape d'agrégation pour former des microsphères polymères, et
le traitement desdites microsphères de polymères pour fixer un matériau fonctionnel biomédical auxdites microsphères polymères,
où lesdites microsphères polymères ont un diamètre de particule moyen dans la plage de 1 à 15 microns avec une distribution de taille géométrique de particule étroite.

2. Procédé selon la revendication 1, dans lequel ladite distribution de taille géométrique de particule étroite est inférieure à 1,25.

3. Procédé selon la revendication 1, dans laquelle lesdites microsphères polymères sont formées à partir d'un polymère sélectionné parmi le groupe constitué des polyesters et de polymères à base d'acrylique.

4. Procédé selon la revendication 3, dans lequel ledit polyester est obtenu à partir d'une estérification par fusion d'acide dicarboxylique ou de composants diester avec des composants diol en présence d'un catalyseur de polycondensation.

5. Procédé selon la revendication 1, dans lequel le matériau fonctionnel biomédical est fixé auxdites microsphères polymères par au moins l'un d'une liaison covalente, d'une formation de complexe, d'une adsorption physique et d'une absorption physique.

6. Procédé selon la revendication 1, dans lequel les microsphères polymères sont formées à partir desdites variétés monomères de précurseur et d'un ou plusieurs additifs.

7. Procédé selon la revendication 1, comprenant en outre un traitement de surface desdites microsphères polymères à la suite de ladite étape de formation mais avant ladite étape de traitement pour modifier une propriété chimique d'une surface desdites microsphères polymères.

8. Procédé selon la revendication 7, dans lequel ladite propriété chimique est sélectionnée parmi le groupe constitué de l'hydrophobie, de la charge de surface et de la présence de groupement fonctionnel.

9. Procédé selon la revendication 1, dans lequel les microsphères polymères sont biocompatibles.

10. Procédé selon la revendication 1, dans lequel les microsphères polymères sont biodégradables.

11. Procédé selon la revendication 1, dans lequel les microsphères polymères sont non biodégradables.

12. Procédé selon la revendication 1, dans lequel l'étape d'agrégation comprend
l'agrégation de ladite résine de polymère en particules polymères,
l'union desdites particules polymères en microsphères polymères, et
éventuellement l'isolement desdites microsphères polymères.

13. Procédé selon la revendication 1, dans lequel l'étape d'agrégation comprend la formation d'une émulsion comprenant ladite résine polymère,
l'union desdites microsphères polymères, et
éventuellement l'isolement desdites microsphères polymères.

14. Procédé selon la revendication 1, dans lequel la résine polymère est une résine polyester, avant l'agrégation la résine polyester est dispersée dans un milieu aqueux comprenant éventuellement un tensioactif, afin de fournir une suspension de particules en suspension de ladite résine polyester et la suspension est homogénéisée, et où l'étape d'agrégation comprend l'agrégation et la réunion de ladite suspension homogénéisée en ajoutant un sel de métal cationique et éventuellement des additifs, et le chauffage des agrégats afin de former des microsphères polymères où ledit chauffage est conduit à une température de transition vitreuse de la résine polyester ou à une température proche de celle-ci.
